# EUROPÄISCHE PATENTSCHRIFT

(11) **EP 1 649 356 B1**
(45) Veröffentlichungstag und Bekanntmachung des Hinweises auf die Patenterteilung: **12.04.2017**
(21) Anmeldenummer: 04763602.2
(22) Anmeldetag: 29.07.2004
(51) Int. Cl.: G06F 3/14, G06F 1/16, G05B 19/042, G06F 19/00

(54) **ANZEIGE- UND STEUERVORRICHTUNG FÜR MEDIZINTECHNISCHE GERÄTE**
DISPLAY AND CONTROL DEVICE FOR MEDICAL EQUIPMENT
DISPOSITIF D'AFFICHAGE ET DE COMMANDE POUR APPAREILS MEDICO-CHIRURGICAUX

(30) Priorität: 29.07.2003 DE 10334516
(43) Veröffentlichungstag der Anmeldung: 26.04.2006
(60) Teilanmeldung: 12175890.8
(73) Patentinhaber: Sorin Group Deutschland GmbH, 80939 München (DE)
(72) Erfinder: KNOTT, Erwin, 85586 Poing (DE); PENKA, Ottmar, 81245 München (DE); Hahn, Andreas, 82335 Berg (DE)
(74) Vertreter: Hoffmann Eitle
(86) Internationale Anmeldenummer: PCT/EP2004/008499
(87) Internationale Veröffentlichungsnummer: WO 2005/013113

(56) Entgegenhaltungen:
- EP-A- 1 246 052
- US-A1- 2003 067 437
- US-B2- 6 496 359
- ANONYMOUS: 'SIII System', [Online] 01 August 2001, XP055082779 Stöckert Instrumente GmbH, München Gefunden im Internet: <URL:http://www.perfusion.com/cgi-bin/absol utenm/articlefiles/stockertSIII/StockertSII I_Manual.pdf> [gefunden am 2013-10-07]

## Beschreibung

Die vorliegende Erfindung betrifft eine Anzeige- und Bedienvorrichtung für medizintechnische Geräte, insbesondere Lebenserhaltungssysteme wie Herz-Lungen-Maschinen oder Beatmungsgeräte.

Um medizintechnische Geräte sicher bedienen und überwachen zu können, müssen an diesen Geräten Anzeige- und Bedienvorrichtungen vorgesehen werden, die es dem Benutzer gestatten, in übersichtlicher Form den Betriebszustand des Geräts zu erfassen und mit Hilfe von Bedienelementen in dem Betrieb des Geräts einzugreifen. So sind zum Beispiel bei Herz-Lungen-Maschinen (im folgenden auch: HLM) Anzeige- und Bedienelemente für die verschiedenen Einheiten und Komponenten, z.B. die Blutpumpen oder den Oxygenator vorgesehen, über die der Benutzer den Betrieb der Einheiten der HLM überwachen und beeinflussen kann. Daneben sind Anzeige- und Bedienelemente für verschiedene Sensoren, beispielsweise Füllstandssensoren, Temperatursensoren oder Luftblasendetektoren vorgesehen, an denen der Benutzer einen Sensormesswert ablesen und Grenzwerte einstellen kann, bei deren Über- bzw. Unterschreiten ein Alarm oder eine andere Aktion ausgelöst wird. Bei HLM werden diese Anzeige- und Bedienelemente neuerdings oft in Form eines Anzeige- und Bedienpaneels zusammengefasst, so dass der Benutzer an einem Ort in übersichtlicher Weise den Betrieb der gesamten Herz-Lungen-Maschine überwachen und steuern kann.

Das S3-System der Stöckert Instrumente GmbH München, zum Beispiel beschrieben in den Operating Instructions, zeigt eine Anzeige- und Steuervorrichtung, zum Beispiel für ein modulares Perfusionssystem, bei dem ein Anzeige- und Bedienpanel vorgesehen ist, an das sechs oder zehn vorab konfigurierte Module installiert werden können.

Anzeige- und Bedienpaneele der hier angesprochenen Art werden in zunehmendem Masse unter Verwendung programmgesteuerter Bildschirme und Tastatureinheiten realisiert. In jüngster Zeit werden diese Benutzerschnittstellen (GUI = Graphical User Interface) in Form von LCD-Anzeigegeräten in Kombination mit einer berührungsempfindlichen Abdeckung der LCD-Anzeigeoberfläche verwendet (Touch Screen). Das bedeutet, der Benutzer kann nicht nur Werte auf der Anzeige ablesen, sondern auch auf der Anzeige bildhaft dargestellte Taster, Schalter und Regler betätigten, indem er die Anzeigenoberfläche im Bereich der Darstellung eines Tasters, Schalters oder Reglers berührt.

Der Einsatz derartiger Touch-Screen-GUI-Einheiten ermöglicht eine einfache Bedienung auch komplexer Systeme, insbesondere durch Reduktion der Bedienelemente und dadurch gesteigerte Übersichtlichkeit, durch kontext-abhängige Gestaltung der Bedienmöglichkeiten und durch situationsabhängige Hilfestellungen durch das System. Auch fertigungsseitig bieten die GUI-Einheiten dieser Art Vorteile, insbesondere bei höheren Stückzahlen. Jedoch ist nachteilig, dass bei Verwendung einer GUI-Einheit ein Anzeigendefekt zu einem vollständigen Verlust der Systemkontrolle führen kann. Eine Interaktion über die GUI-Einheit mit dem Benutzer ist bei einem Totalausfall nicht mehr möglich. Auch Teilausfälle können zu einer nicht akzeptablen Beeinträchtigung der Bedienbarkeit des medizintechnischen Gerät führen. Dabei ist zu beachten, dass berührempfindliche Oberflächen systembedingt anfällig für mechanische Defekte sind. Bei komplexen Anwendungen ist überdies ein aufwendiger Entwicklungsprozess für die GUI-Einheit und deren Ansteuerung erforderlich, insbesondere bei der Realisierung sprachspezifischer Varianten.

Dies hat zur Folge, dass derartige Anzeige- und Bedienvorrichtungen für sicherheitskritische Anwendungen in Lebenserhaltungssystemen, wie Herz-Lungen-Maschinen oder Beatmungsgeräten nur mit erheblichem Aufwand (redundante Bedienfelder, speziell angepasste Betriebssysteme etc.) eingesetzt werden können. Wo eine redundante Auslegung nicht möglich oder sinnvoll ist, muss die Anzeige- und Bedienvorrichtung als Austauschteil bereitgestellt werden, das bei Versagen der eingesetzten Anzeige- und Bedienvorrichtung gegen diese ausgetauscht werden kann. Im Hinblick auf die Kosten für die Bereitstellung eines Austauschpaneels ist diese Vorgehensweise doch unbefriedigend.

US 6.496.359 B2 beschreibt modulare Computer, die jeweils neben dem die Rechenleistung bereitstellenden Prozessor und einem Speicher auch ein flaches Anzeigepaneel mit berührungsempfindlicher Oberfläche aufweisen. Die Computermodule sind autark und stellen jeweils für sich einen Rechner mit berührungsempfindlicher Anzeige dar. Die Computermodule können an einem Panel befestigt werden, das die Stromversorgung der Computermodule bereitstellt und auch die Kommunikation mit den Computermodulen von außen, beispielsweise die Kommunikation mit einem externen Server, ermöglicht. Die Computermodule können und sollen aber daneben auch untereinander kommunizieren, um zusammen agieren zu können. Durch die Modularität und Interaktionsfähigkeit wird die Möglichkeit geschaffen, Rechenleistung und Anzeigefläche zu größeren Einheiten zusammenzufassen, indem mehrere Computermodule verbunden werden, so dass dadurch eine adäquate Rechenleistung und eine zusammenhängende Anzeigefläche geschaffen wird.

Vor diesem Hintergrund hat die Erfindung zum Ziel eine Anzeige- und Bedienvorrichtung für medizintechnische Geräte, insbesondere Herz-Lungen-Maschinen oder andere Lebenserhaltungssysteme wie etwa Beatmungssysteme anzugeben, bei der im Falle eines Defekts die Fortsetzung des Betriebs auf wirtschaftliche Weise gewährleistet werden kann.

Dieses Ziel wird erreicht durch eine Anzeige- und Bedienvorrichtung mit den Merkmalen des Patentanspruchs 1. Vorteilhafte Ausgestaltungen ergeben sich aus den Unteransprüchen.

Erfindungsgemäß werden mehrere Touch-Screen-GUI-Einheiten zu einer modularen Anzeige- und Bedienvorrichtung zusammengesetzt. Die GUI-Einheiten sind in ihrem Aufbau praktisch identisch und als Steckmodul ausgeführt, das an verschiedenen Positionen eines Basispaneels angebracht werden kann. Die Festlegung der Anzeige- und Bedienfunktion der einzelnen Touch-Screen-GUI-Einheit erfolgt durch ein Konfigurationseinrichtung, mit der die Touch-Screen-GUI-Einheit über ein Bussystem (in der Basis) verbunden ist und von der sie Konfigurationsdaten erhält. Die Konfigurationsdaten bestimmen über die Anzeigeninhalte und Eingabebereiche die Anzeige- und Bedienfunktion der Touch-Screen-GUI-Einheit.

Im folgenden wird die Erfindung anhand eines Ausführungsbeispiels unter Bezugnahme auf die Zeichnungen genauer erläutert, in denen zeigt:
- Fig. 1: schematisch den Aufbau einer HLM als ein für die Erläuterung der Erfindung besonders geeignetes Beispiel eines Lebenserhaltungssystems im Speziellen und eines medizintechnischen Geräts im Allgemeinen;
- Fig. 2: eine perspektivische Darstellung des Aufbaus einer erfindungsgemäßen Anzeige- und Bedienvorrichtung;
- Fig. 3: eine perspektivische Darstellung der Vorderseite einer Anzeige/Bedieneinheit gemäß der Erfindung;
- Fig. 4: eine perspektivische Darstellung der Rückseite einer Anzeige/Bedieneinheit gemäß der Erfindung;
- Fig. 5: eine perspektivische Darstellung des Aufbaus einer Basiseinheit gemäß der Erfindung;
- Fig. 6: eine Darstellung einer Basiseinheit und einer daran angeordneten Anzeige/Bedieneinheit gemäß der Erfindung;
- Fig. 7A bis 7E: Beispiele für auf Konfigurationsdaten zurückgehende Anzeigeninhalte einer Anzeige/Bedieneinheit gemäß der Erfindung.

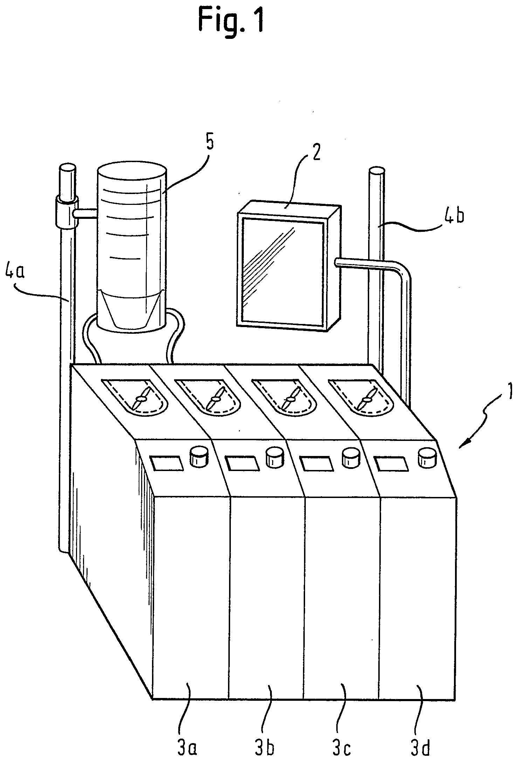

In Fig. 1 ist schematisch eine Herz-Lungen-Maschine 1 dargestellt, an der exemplarisch eine Anzeige- und Bedienvorrichtung 2 erläutert werden soll, wie sie bei medizintechnischen Geräten, insbesondere Lebenserhaltungssystemen wie Herz-Lungen-Maschinen und Beatmungsgeräten anzutreffen ist.

Die in Fig. 1 gezeigte Herz-Lungen-Maschine 1 umfasst vier Blutpumpen 3a, 3b, 3c und 3d, die nebeneinander angeordnet sind. Die Gehäuse der Blutpumpen bilden den Grundkörper der HLM 1. An Befestigungsmasten 4a und 4b können weitere Aggregate oder Einheiten der HLM 1 angeordnet sein, wie beispielsweise das schematisch angedeutete Blutreservoir 5. Überdies sind bei einer HLM üblicherweise Sensoren (nicht dargestellt) vorhanden, um verschiedene Messwerte wie Temperaturen, Drücke und Flüsse zu erfassen. Ferner sind üblicherweise Blasendetektoren (nicht dargestellt) vorgesehen, über die sichergestellt wird, dass dem Patienten kein Luftblasen enthaltendes Blut zugeführt wird. Es ist nicht beabsichtigt, die Einheiten oder Komponenten einer HLM hier vollständig aufzuzählen. Die zuvor erwähnten Einheiten sind lediglich exemplarisch anhand einer HLM, aber auch für die übrigen Lebenserhaltungssysteme bzw. medizintechnischen Geräte erwähnt. Jedoch eignet sich eine HLM ebenso wie Beatmungsgeräte in besonderem Maße für die Anwendung der Erfindung.

An der in Fig. 1 gezeigten HLM 1 ist die bereits angesprochene Anzeige- und Bedienvorrichtung 2 vorgesehen, mit der die Einheiten oder Komponenten der HLM 1 überwacht und gesteuert werden können. Dazu zeigt die Anzeige- und Bedienvorrichtung 2 verschiedene Werte an, teilweise Messwerte der Sensoren und Detektoren, teilweise Betriebsparameter der Einheiten, wie beispielsweise die Drehzahl der Blutpumpen oder auch andere Werte, beispielsweise Zeiten. Der Benutzer erhält über die Anzeige der Werte einen Überblick über den Betriebszustand der HLM 1 und kann über die Anzeige- und Bedienvorrichtung 2 steuernd eingreifen, indem er die in der Anzeige- und Bedienvorrichtung bildhaft angebotenen Bedienelemente benutzt.

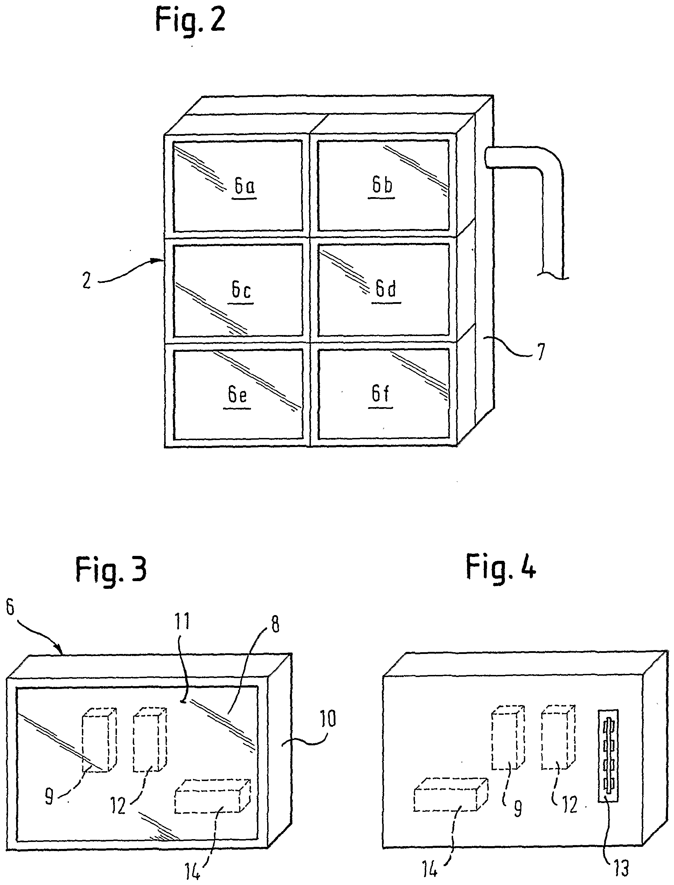

In Figur 2 ist eine erfindungsgemäße Anzeige- und Bedienvorrichtung 2 dargestellt; sie umfasst mehrere Anzeige-/Bedieneinheiten 6a bis 6f, die auf einer Basiseinheit 7 angeordnet sind. Wie Figur 3 zeigt, in der eine Vorderansicht einer erfindungsgemäßen Anzeige-/Bedieneinheit 6 dargestellt ist, umfasst jede Anzeige-/Bedieneinheit 6 eine flache Anzeigeeinrichtung 8, die eine Vielzahl von ansteuerbaren Bildpunkten aufweist und auf der sowohl Messwerte in Form von Zahlenwerten als auch Text oder grafische Bestandteile angezeigt werden können. Dazu ist die Anzeige-/Bedieneinheit 6 mit einer Anzeigesteuereinrichtung 9 ausgestattet, die die Bildpunkte der Anzeigeeinrichtung 8 auf der Grundlage von zugeführten Daten ansteuert. Die Anzeigesteuereinrichtung 9 ist vorzugsweise in einem Gehäuse 10 der Anzeige-/Bedieneinheit 6 vorgesehen, weshalb sie in der Darstellung der Figur 3 gestrichelt wiedergegeben ist. Um eine Eingabemöglichkeit zu schaffen weist jede Anzeige-/Bedieneinheit eine transparente Eingabeeinrichtung 11 auf, die auf der einem Betrachter zugewandten Oberfläche der Anzeigeeinrichtung 8 angeordnet ist und die mit einer Eingabeauswerteeinrichtung 12 in Verbindung steht, die die über die Eingabeeinrichtung 11 erfolgenden Eingaben auswertet. Auch die Eingabeauswerteeinrichtung 12 ist vorzugsweise in dem Gehäuse 10 untergebracht und deshalb gestrichelt dargestellt. Insgesamt wird auf diese Weise eine Touch-Screen-Anzeige/Eingabeeinheit 6 geschaffen, die einerseits Informationen anzeigen (8, 9) und andererseits Eingaben entgegennehmen (11, 12) kann. Weitere Anzeige- oder Bedienelemente sind erfindungsgemäß nicht erforderlich, können aber vorgesehen werden. Insbesondere kann ein Ein/Aus-Schalter vorgesehen werden, damit beim Aufstecken der Anzeige/Bedieneinheit 6 auf die Basiseinheit 7, die Anzeige/Bedieneinheit 6 nicht aktiv ist.

Damit die Daten für die Anzeigeninhalte zu Anzeigesteuereinrichtung 9 übertragen und die Eingaben von der Eingabeauswerteeinrichtung 12 weitergeleitet werden können, umfasst die Anzeige/Bedieneinheit 6 eine Anschlusseinrichtung 13, die in Figur 4 - einer Rückansicht einer Anzeige/Bedieneinheit - gezeigt ist. Mit der Anschlusseinrichtung 13 sind die Anzeigesteuereinrichtung 9 und die Eingabeauswerteeinrichtung 12 verbunden, so dass die erfindungsgemäße Anzeige/Bedieneinheit 6 an einen elektrischen Bus anschließbar ist.

Bei dem hier angesprochenen elektrischen Bus handelt es sich um ein Verbindungssystem, das es an den Bus angeschlossenen elektrischen Geräten erlaubt, mit anderen an den Bus angeschlossenen Geräten zu kommunizieren. Das bedeutet im wesentlichen, dass Werte oder andere Informationen gezielt von einem Busgerät zu einem anderen Busgerät übertragen, d.h. gesendet und empfangen werden können.

In Abhängigkeit von der Art des eingesetzten Busses kann zweckdienlicherweise eine Buskommunikationseinrichtung 14 vorgesehen werden, die einerseits die Verbindung zum Bus gewährleistet und andererseits mit der Anzeigesteuereinrichtung 9 und der Eingabeauswerteeinrichtung 12 verbunden ist, wodurch die Anzeigesteuereinrichtung 9 und die Eingabeauswerteeinrichtung 12 nicht für einen direkten Anschluss an den Bus ausgelegt werden müssen.

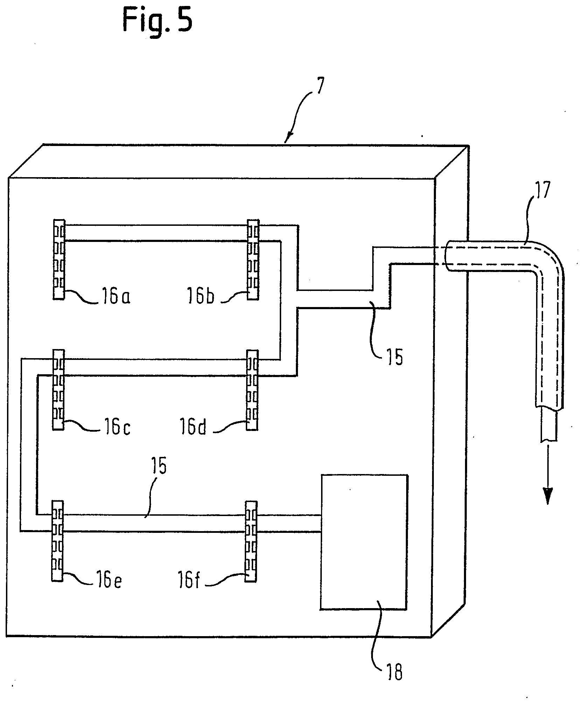

Wie Figur 5 zeigt, ist der Bus 15 in der Basiseinheit 7 der erfindungsgemäßen Anzeige- und Bedienvorrichtung 2 vorgesehen. Er dient, wie bereits ausgeführt, der Kommunikation von an den Bus angeschlossenen Einheiten. Damit die Anzeige/Bedieneinheiten 6a bis 6f an den Bus angeschlossen werden können, sind an der Basiseinheit 7 geeignete Verbindungseinrichtungen 16a bis 16f vorgesehen. Jede Verbindungseinrichtung 16 in der Basiseinheit 7 ist an die Anschlusseinrichtung 13 der Anzeige-/Bedieneinheiten 6 angepasst, so dass durch Zusammenführen einer Anschlusseinrichtung 13 und einer Verbindungseinrichtung 16, vorzugsweise nach dem Stecker-Buchse-Prinzip, eine Anzeige/Bedieneinheit 6 an den Bus 15 anschließbar ist, wenn die Anzeige/Bedieneinheit 6 an der Basiseinheit 7 angeordnet wird.

Die Verbindung zwischen Verbindungseinrichtung 16 und Anschlusseinrichtung 13 ist entweder derart, dass auch eine ausreichende mechanische Halterung der Anzeige/Bedieneinheit 6 an der Basiseinheit 7 erreicht wird, oder derart, dass zusätzliche Vorkehrungen zur mechanische Halterung getroffen werden müssen, die hier aber nicht näher erläutert werden. Im Grunde eignet sich jede von einem Benutzer einfach herstellbare und wieder lösbare Halterung der Anzeige/Bedieneinheit 6 an der Basiseinheit 7, wobei die Anforderungen zu beachten sind, die im Umfeld der medizintechnischen Geräte und insbesondere der Lebenserhaltungssysteme existieren.

Der Bus 15 ist aus der Basiseinheit 7 herausgeführt, beispielsweise, wie in Figur 5 gezeigt, durch die Stütze 17 der Anzeige- und Bedienvorrichtung 2. Durch die Stütze 17 führt der Bus in das medizintechnische Geräte, wie beispielsweise bei der HLM in Figur 1 erkennbar, so dass die Einheiten und Aggregate des medizintechnischen Geräts auch an den Bus angeschlossen werden können.

In der Basiseinheit 7 ist ferner eine Konfigurationseinheit 18 vorgesehen, die auch mit dem elektrischen Bus 15 verbunden ist. Die Konfigurationseinheit 18 übernimmt unmittelbar nach dem Anschließen einer Anzeige/Bedieneinheit 6 an den Bus, also in der Regel beim Anstecken der Anzeige/Bedieneinheit 6 an die Basiseinheit 7, die Aufgabe, der Anzeige/Bedieneinheit 6 erste Daten zu übermitteln. Dabei handelt es sich um Konfigurationsdaten, die der Anzeige/Bedieneinheit 6 mitteilen, welche Aufgabe sie in der Gesamtheit der Anzeige- und Bedienvorrichtung 2 zu übernehmen hat. Die Konfiguration der Anzeige/Bedieneinheit 6 ist ein wichtiger Aspekt der erfindungsgemäßen Anzeige- und Bedienvorrichtung 2, da dadurch eine Anzeige/Bedieneinheit 6 unterschiedlich verwendet werden kann, da erst die Konfigurationsdaten festlegen, wofür die Anzeige/Bedieneinheit 6 eingesetzt wird. Im wesentlichen handelt es sich bei den über den elektrischen Bus 15 übermittelten Konfigurationsdaten um Informationen (Anweisungen), die die Anzeigeninhalte und die Eingabebereiche der Anzeige-/Bedieneinheit 6 festlegen. Die Anzeigesteuereinrichtung 9 der Anzeige/Bedieneinheit 6 muss die durch die Konfigurationsdaten übermittelten Anzeigeinhalte in der Anzeige 8 der Anzeige/Bedieneinheit 6 darstellen (Figur 3); die Eingabeauswerteeinrichtung 12 muss dementsprechend die durch die Konfigurationsdaten als Eingabebereiche definierten Abschnitte auf der Oberfläche 11 der Anzeige 8 auswerten und auf Eingaben durch den Benutzer überwachen.

Da die Anzeige/Bedieneinheiten 6 einer erfindungsgemäßen Anzeige- und Bedienvorrichtung 2 praktisch identisch aufgebaut sind, kann jede Anzeige/Bedieneinheit 6 an jeder Stelle (16a bis 16f) der Basiseinheit 7 eingesetzt werden. Das bedeutet, dass während der Benutzung des medizintechnischen Geräts, beispielsweise der HLM im Operationssaal in der Regel nur eine Anzeige/Bedieneinheit als Austauschteil vorgehalten werden muss, da bei Ausfall irgendeiner Anzeige/Bedieneinheit in der Anzeige- und Bedienvorrichtung die redundante Anzeige/Bedieneinheit als Ersatz eingesetzt werden kann. Darüber hinaus kann eine bereits verwendete Anzeige/Bedieneinheit außer Betrieb genommen werden, wenn ihre Funktion von untergeordneter Bedeutung ist (z.B. Zeitanzeige) und an die Stelle einer ausgefallenen Anzeige/Bedieneinheit gesetzt werden, die kritische Überwachungs- Steuerungsfunktionen unterstützt, die für den Benutzer unverzichtbar sind.

Neben der Möglichkeit eine Anzeige/Bedieneinheit als kostengünstiges Austauschteil bereitzuhalten, schafft die Erfindung die Möglichkeit, durch redundante Auslegung der Konfigurationseinrichtung 18 einen sicheren Betrieb zu gewährleisten. Auf die redundante Auslegung der Konfigurationseinrichtung 18 stellt einen akzeptablen Aufwand dar, wodurch die Erfindung ermöglicht wird. Daneben ist zu beachten, dass während des Betriebs der Anzeige- und Bedienvorrichtung gemäß der Erfindung die Konfigurationseinrichtung 18 keine weiteren Funktionen übernimmt. Das bedeutet, dass ihr Ausfall während des Betriebs nach abgeschlossener Konfiguration der Anzeige/Bedieneinheiten zunächst keine Beeinträchtigung der Gesamtfunktion der Anzeige- und Bedienvorrichtung gemäß der Erfindung darstellt. Dennoch sollte eine Störung der Konfigurationseinrichtung 18 dem Benutzer signalisiert werden, da bei gestörter Konfigurationseinrichtung 18 ein Austausch einer Anzeige/Bedieneinheit 6 nicht mehr möglich ist.

Im folgenden wird beispielhaft beschrieben, in welcher Form Konfigurationsdaten von der Konfigurationseinrichtung 18 an eine Anzeige/Bedieneinheit 6 übertragen werden und in welcher Art und Weise die Konfigurationsdaten in der Anzeige/Bedieneinheit 6 verarbeitet werden.

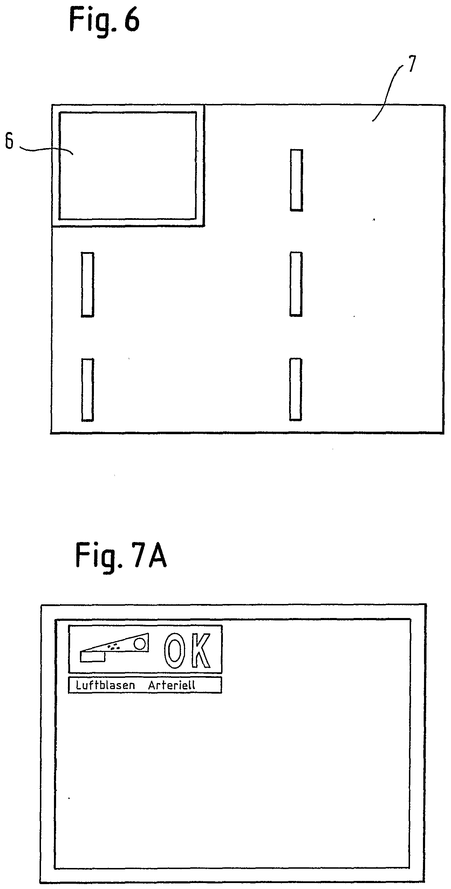

Dazu zeigt Figur 6 eine Basiseinheit 7, an der eine Anzeige/Bedieneinheit 6 angesteckt ist. Wenn eine Anzeige/Bedieneinheit 6 an der Basiseinheit 7 angeordnet und durch Zusammenführen der Anschlusseinrichtung 13 und der Verbindungseinrichtung 16 an den elektrischen Bus 15 in der Basiseinheit 7 angeschlossen wird, wird der Konfigurationseinrichtung 18 über den Bus 15 signalisiert, dass und an welcher Verbindungseinrichtung 16a bis 16f eine Anzeige/Bedieneinheit 6 angeschlossen wurde. Da die Position der Verbindungseinrichtung 16a bei dem hier beschriebenen Ausführungsbeispiel vorgibt, welche Anzeigeninhalte dargestellt und welche Eingaben entgegengenommen werden, übermittelt die Konfigurationseinrichtung 18 über den Bus 15 solche Konfigurationsdaten an die Anzeige/Bedieneinheit 6, die der Ansteckposition auf der Basiseinheit 7 der Anzeige- und Bedienvorrichtung 2 entsprechen.

Einzelheiten des Konfigurationsvorgangs wird im Folgenden anhand der Figuren 7A bis E erläutert. Dabei wird der Einsatz einer erfindungsgemäßen Anzeige- und Bedienvorrichtung 2 an einer Herz-Lungen-Maschine (vgl. Figur 1) betrachtet, die neben anderen Einheiten zum Beispiel einen Sensor für die Füllstandsüberwachung einem Blutreservoirs und einen Detektor für Luftblasen in der arteriellen Zuleitung, sowie Temperatursensoren für das in das Blutreservoir eintretende und das aus dem Blutreservoir austretende Blut besitzt. Diese Sensoren und Detektoren, aber auch die anderen Einheiten und Komponenten der HLM sind an den Bus 15 angeschlossen. Die Messwerte dieser Sensoren/Detektoren sollen mit Hilfe einer erfindungsgemäßen Anzeige/-Bedienvorrichtung 2 visualiert werden, wobei beispielhaft festgelegt ist, dass die Darstellung immer im Bereich der Verbindungseinrichtung 16a auf der Basiseinheit 7 der Anzeige- und Bedienvorrichtung 2 erfolgt.

Wenn nun eine Anzeige/Bedieneinheit 6 an der Position 16a der Basiseinheit 7 angeordnet und eine Verbindung zum Bussystem 15 hergestellt wird, übermittelt die Konfigurationseinrichtung 18 der Anzeige/Bedieneinheit 6 Konfigurationsdaten, die die Anzeigeinhalte und die Eingabebereiche festlegen.

In einem ersten Schritt übermittelt die Konfigurationseinrichtung der Anzeige/Bedieneinheit beispielsweise die Konfigurationsdaten:
(1) *DispletPos="1"*
(2) *Title Color="Blau" "Luftblasen Arteriell"*
   und veranlasst die Anzeige/Bedieneinheit 6 in der obersten von drei vordefinierten Zeilen in der linken Hälfte des Anzeigebereichs 8 eine blaue Titelzeile mit dem Text "Luftblasen arteriell" anzuzeigen, was in Figur 7A gezeigt ist. Bei dem Anzeigebereich handelt es sich um eine logische Unterteilung der ansonsten vorteilhafterweise kontinuierlichen und nicht unterteilten Anzeige 8 der Anzeige/Bedieneinheit 6. In einem zweiten Schritt übermittelt die Konfigurationseinrichtung 18 der Anzeige/Bedieneinheit 6 die Konfigurationsdaten
(3) *DispIcon1 Pic="BubbleDetector"*
   und veranlasst die Anzeige/Bedieneinheit 6 oberhalb der Titelzeile "Luftblasen arteriell" ein schematisches Bild für einen Luftblasendetektor anzuzeigen, was auch aus Figur 7A entnehmbar ist. In einem dritten Schritt übermittelt die Konfigurationseinrichtung 18 der Anzeige/Bedieneinheit 6 die Konfigurationsdaten
(4) *ValueCan="0000062"*
   die die Anzeige/Bedieneinheit 6 veranlassen, Werte des Busgeräts mit der Identifikation 000062 über den elektrischen Bus 15 entgegenzunehmen. Diese Identifikation ist beispielsweise dem Luftblasendetektor der HLM zugewiesen worden, so dass die Anzeige/Bedieneinheit 6 durch die übermittelten Konfigurationsdaten dafür konfiguriert wird, Sensorsignale des Luftblasendetektors entgegenzunehmen. In einem vierten Schritt übermittelt die Konfigurationseinrichtung 18 der Anzeige/Bedieneinheit 6 die Konfigurationsdaten
(5) *ValIcon2 Pic="OK" Stat<"10"*
(6) *ValIcon2 Pic="Alarm" Stat>="10"*
   und veranlasst damit die Anzeige/Bedieneinheit 6 die Buchstaben "OK" in dem Bereich über der Titelzeile "Luftblasen arteriell" anzuzeigen, wenn der von den Luftblasendetektor erhaltene Wert kleiner als 10 ist. Wenn der Wert größer oder gleich 10 ist, enthalten die Konfigurationsdaten das Wort "ALARM", dass die Anzeige/Bedieneinheit 6 anstelle der Buchstaben "OK" anzeigt. In Figur 7A ist die Anzeige "OK" dargestellt.

Um die Anzeigeinhalte in der rechten Hälfte der obersten Zeile der Anzeige/Bedieneinheit 6 zu festzulegen, übermittelt die Konfigurationseinrichtung 18 an die Anzeige/Bedieneinheit 6 die folgenden Konfigurationsdaten
(7) *Displet="2"*
(8) *Title Color="Grün" "Reservoir Level"*
(9) *ValueCan="000061*"
(10) *ValIcon1 Pic="FullRes" Stat>"75"*
(11) *ValIcon1 Pic="75%Res" Stat>"50"*
(12) *ValIcon1 Pic="50%Res" Stat>"25"*
(13) *ValIcon1 Pic=*"*25*%*Res*" *Stat>"5"*
(14) *ValIcon1 Pic="0%Res" Stat>"0"*
(15) *ValIcon2 Pic="OK" Stat>"25"*
(16) *ValIcon2 Pic="LOW" Stat<="25"*

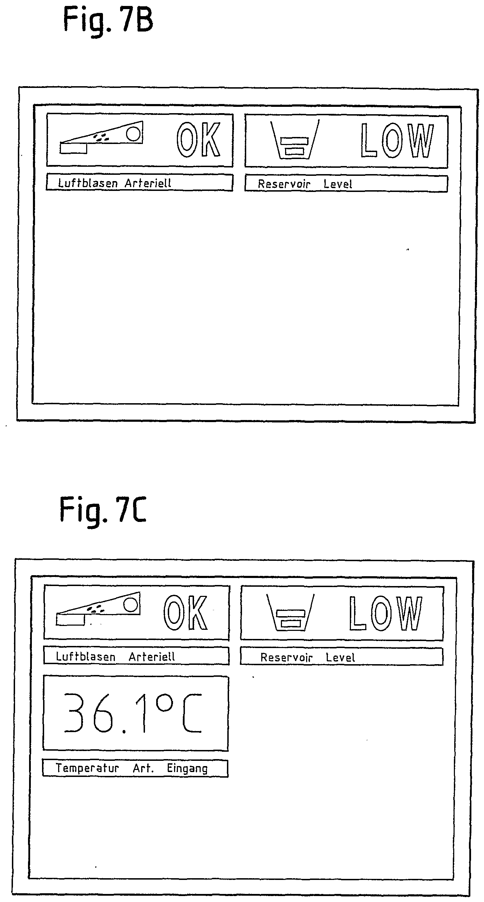

Das Ergebnis dieser Konfigurationsdaten ist in Figur 7B gezeigt, das sich wie folgt ergibt. In den Konfigurationsdaten legt Zeile (7) fest, dass das Anzeigesegment in der rechten Hälfte der obersten Zeile der Anzeige/Bedieneinheit 6 angesprochen wird. Durch die Konfigurationsdaten in Zeile (8) wird veranlasst, dass die Anzeige/Bedieneinheit 6 eine Titelzeile in Grün mit dem Titel "Reservoir Level" anzeigt.

Die Konfigurationsdaten in Zeile (9) veranlassen die Anzeige/Bedieneinheit 6, Daten der an den elektrischen Bus angeschlossenen Einheit mit der Identifikation 000061 entgegenzunehmen. Bei dem hier beschriebenen Ausführungsbeispiel handelt es sich dabei um den Füllstandssensor, der den Füllstand des Blutreservoirs der hier betrachteten HLM als Wert über den elektrischen Bus 15 kommuniziert.

Die Konfigurationsdaten in den Zeilen (10) bis (14) veranlassen die Anzeige/Bedieneinheit 6 in Abhängigkeit von den empfangenen Daten des Füllstandssensors unterschiedliche Grafiken anzuzeigen, die den Füllstand des Reservoirs wiederspiegeln. Dabei werden die unterschiedliche Grafiken ausgewählt in Abhängigkeit von den Stufen 75%, 50%, 25% und 5%.

Die Konfigurationsdaten in den Zeilen (15) und (16) veranlassen die Anzeige/Bedieneinheit 6 ferner die Buchstaben "OK" im oberen rechten Bereich der Anzeige darzustellen, wenn der Wert des Füllstandssensors größer als 25 ist. Die Buchstaben "OK" werden durch das Wort "LOW" ersetzt, wenn die Werte des Füllstandssensors kleiner oder gleich 25 sind.

Die folgenden Konfigurationsdaten, deren Anzeigeergebnis in Figur 7C gezeigt ist, veranlassen die Anzeige/Bedieneinheit 6 in der linken Hälfte der zweiten Zeile de Anzeige 8 eine Titelzeile in gelber Farbe mit der Bezeichnung "Temperatur Art. Eingang" darzustellen und darüber einen Temperaturwert mit drei Stellen und Dezimalpunkt und die Einheit "°C", wobei die darzustellenden Werte von einem Temperatursensor mit der Identifikation 000060 stammen.
(17) *Displet="3"*
(18) *Title Color="Gelb" "Temperatur Art. Eingang"*
(19) *ValueCan="000060"*
(20) *Value Fmt="%3d" Unit="°C"*

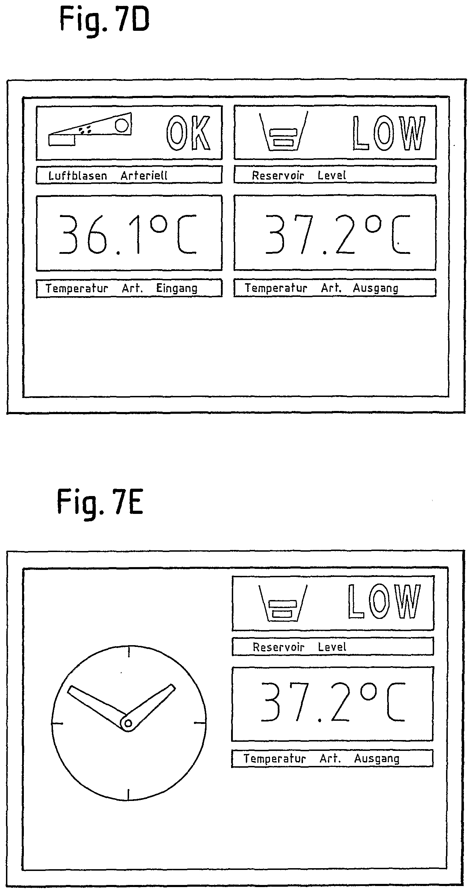

Entsprechend veranlassen die folgenden Konfigurationsdaten eine Anzeige in der rechten Hälfte der zweiten Zeile des Anzeige 8 der Anzeige/Bedieneinheit 6, wie Figur 7D zeigt, wobei die hier darzustellenden Werte von einem Temperatursensor mit der Identifikation 000059 stammen.
(21) *Displet="4"*
(22) *Title Color="Gelb" "Temperatur Art. Ausgang"*
(23) *ValueCan="000059"*
(24) *Value Fmt=*"%*3d*" *Unit="°C"*

Die zuvor beschriebenen Konfigurationsdaten veranlassen die Anzeige/Bedieneinheit 6 ferner, den Bereich der Anzeige oberhalb der Titelzeilen als Eingabebereiche festzulegen. Das bedeutet, dass wenn ein Benutzer die Oberfläche der Anzeige 8 der Anzeige/Bedieneinheit 6 im Bereich oberhalb einer der Titelzeilen berührt wird, eine Eingabe zu dem betreffenden Sensor entgegengenommen. Wenn beispielsweise aufgrund eines zu geringen Füllstands im Blutreservoir (vgl. Figur 7B) ein Alarm ausgelöst wurde, kann der Eingabebereich oberhalb der Titelzeile "Reservoir Level" ausgewertet werden, so dass eine Berührung dieses Bereichs durch den Benutzer als Bestätigung des Alarms angesehen wird.

In den Konfigurationsdaten können auch solche Daten vorgesehen werden, die Eingabebereiche ohne die Festlegung von Messwerteanzeigen bestimmen. Auf diese Weise können Schalter, Taster oder Regler dargestellt werden, die durch Berührung der Berührungsempfindlichen Oberfläche der Anzeige/Bedieneinheit 6 betätigt werden.

Die verschiedenen Bereiche innerhalb der Anzeige einer Anzeige/Bedieneinheit 6 können auch verbunden werden, um größere zusammenhängende Anzeigenbereiche zu bilden. So zeigt zum Beispiel Fig. 7E eine konfigurierte Anzeige/Bedieneinheit, bei der die drei übereinander liegenden Bereiche in der linken Hälfte der Anzeige 8 zu einem Anzeigenbereich zusammengefasst wurden, um einen Anzeigenbereich zu schaffen, der die Darstellung einer analogen Uhr aufnehmen kann. Die Konfigurationsdaten, die die Konfigurationseinrichtung der Anzeige/Bedieneinheit 6 für diesen Anzeigeninhalt übermittelt, haben beispielhaft folgendes Format:
(25) *Displet="1,3,5"*
(26) *DisplIcon Pic="ClockAnalog"*

Die von der Anzeigesteuereinrichtung 9 auf der Anzeige 8 der erfindungsgemäßen Anzeige/Bedieneinheit 6 darzustellenden Anzeigeninhalte sind vorzugsweise in einem Speicher der Anzeigesteuereinrichtung 9 abgelegt. Im wesentlichen handelt es sich dabei um Ziffern und Buchstaben, sowie grafische Bildelemente, die Messwerte oder Betriebszustände, aber auch die Einheiten und Komponenten des medizintechnischen Geräts repräsentieren. Die Abspeicherung dieser Daten in der Anzeigesteuereinrichtung 9 führt dazu, dass von der Konfigurationseinrichtung 18 keine Daten für die Darstellung der Ziffern, Buchstaben und grafischen Bilddarstellungen übermittelt werden müssen. Diese Ausgestaltung einer erfindungsgemäßen Anzeige- und Bedienvorrichtung ist vorteilhaft, jedoch kann bei Steuerungssystemen auch eine Übertragung der Daten für die Anzeigeninhalte von der Konfigurationseinrichtung vorgesehen werden. In jedem Fall ist es vorteilhaft, wenn die Anzeigesteuereinrichtung 9 in der Lage ist, zusätzliche Daten für Anzeigeninhalte zu empfangen, in einem Speicher abzulegen und während des Betriebs für die Darstellung der Anzeige 8 der erfindungsgemäßen Anzeige/Bedieneinheit 6 zu verwenden. Dies erweitert die Einsetzbarkeit einer erfindungsgemäßen Anzeige/Bedieneinheit 6, da bei der Herstellung unbekannter Anzeigeninhalte nachträglich von der Konfigurationseinrichtung 18 an die Anzeige/Bedieneinheit 6 übertragen werden können. Vorzugsweise ist die Anzeigesteuereinrichtung 9 in diesem Fall in der Lage, die Kommunikationseinrichtung 18 über den Bus 15 zu vermitteln, welche Daten bereits in der Anzeigesteuereinrichtung 9 abgespeichert sind, so dass die Konfigurationseinrichtung 18 nur noch solche Daten für die Anzeigeninhalte überträgt, die nicht bereits in der Anzeigesteuereinheit 9 vorhanden sind.

Die hier beschriebene Vorgehensweise für den Aufbau, den Inhalt und das Ergebnis der Konfigurationsdaten sowie die Art des Bussystems dient als Beispiel und wirkt nicht beschränkend auf das Prinzip der erfindungsgemäße Anzeige- und Bedienvorrichtung, die auch mit anderen Konfigurationsdaten und anderen Bussystemen realisierbar ist.

In dem beschriebenen Ausführungsbeispiel bestimmte die Position, an der eine Anzeige/Bedieneinheit 6 an die Basiseinheit 7 und damit an den Bus 15 angeschlossen wurde, welche Funktion der Anzeige/Bedieneinheit zugewiesen wurde. Diese Variante ist vorteilhaft, da auf dem Bedienpaneel Werte und Bedienelemente immer an derselben Stelle vorkommen. Alternativ kann vorgesehen werden, dass Konfigurationsdaten der Reihe nach den Anzeige/Bedienelemente übermittelt werden, sodass der Benutzer festlegen kann, an welcher Stelle des Bedienpaneels welche Funktion bereitgestellt wird.

## Patentansprüche

1. Anzeige- und Bedienvorrichtung für medizintechnische Geräte, die an einen elektrischen Bus (15) anschließbare Einheiten aufweisen, mit:
zumindest einer Anzeige-/Bedieneinheit (6) mit
∘ einer Anzeigeeinrichtung (8)
∘ einer Anzeigesteuereinrichtung (9), die eingerichtet ist, die Anzeigeeinrichtung auf der Grundlage von zugeführten Daten anzusteuern,
∘ einer Eingabeeinrichtung (11)
∘ einer Eingabeauswerteeinrichtung (12), die eingerichtet ist, die über die Eingabeeinrichtung erfolgenden Eingaben auszuwerten und
∘ einer Anschlusseinrichtung (13), mit der die Anzeigesteuereinrichtung (9) und die Eingabeauswerteeinrichtung (12) in Verbindung steht und mit der die Anzeige-/Bedieneinheit (6) an einen elektrischen Bus (15) anschließbar ist und
- einer Basiseinheit (7) mit
∘ einem elektrischem Bus (15) für die Kommunikation von an den elektrischen Bus angeschlossenen Einheiten, an den die Einheiten des medizintechnischen Geräts anschließbar sind,
∘ Verbindungseinrichtungen (16), die derart eingerichtet sind, dass durch Zusammenführen der Anschlusseinrichtung (13) und einer jeweiligen der Verbindungseinrichtungen (16) die Anzeige-/Bedieneinheiten (6) an den elektrischen Bus (15) derart anschließbar sind, dass eine Kommunikation zwischen jeder der Anzeige-/Bedieneinheiten (6) und den an den elektrischen Bus (15) angeschlossenen Einheiten des medizintechnischen Geräts möglich ist, und
∘ einer Konfigurationseinheit (18), die an den elektrischen Bus (15) angeschlossen ist und die eingerichtet ist, der Anzeige- /Bedieneinheit (6) über den elektrischen Bus (15) Konfigurationsdaten zu übermitteln, wobei die Konfigurationsdaten die Anzeigeninhalte der Anzeige-/Bedieneinheit (6) und die für die Kommunikation mit der Anzeige-/Bedieneinheit (6) vorgesehenen Einheiten des medizintechnischen Geräts festlegen;
**gekennzeichnet dadurch, dass**
- die Anzeigeeinrichtung (8) eine Vielzahl von ansteuerbaren Bildpunkten aufweist;
- die Eingabeeinrichtung (11) transparent ist und auf der einem Betrachter zugewandten Oberfläche der Anzeigeeinrichtung (8) angeordnet ist;
- die Konfigurationsdaten auch die Eingabebereiche der Anzeige-/Bedieneinheit (6) festlegen;
- in der Konfigurationseinheit (18) für jede der Verbindungseinrichtungen (16) der Basiseinheit (7) festgelegt ist, welche Konfigurationsdaten zu einer an der jeweiligen Verbindungseinrichtung (16) angeschlossenen Anzeige-/Bedieneinheit (6) übertragen werden; und
- die Konfigurationseinheit (18) eingerichtet ist, um nach dem Anschließen einer Anzeige-/Bedieneinheit (6) an den elektrischen Bus (15) der Anzeige-/Bedieneinheit (6) über den elektrischen Bus (15) die der Verbindungseinrichtung (16), an die die Anzeige-/Bedieneinheit (6) angeschlossen ist, entsprechenden Konfigurationsdaten zu übermitteln.

2. Anzeige- und Bedienvorrichtung nach Anspruch 1, **dadurch gekennzeichnet, dass** in der Anzeige (8) der Anzeige/Bedieneinheit (6) mehrere Bereiche für die Anzeige von Anzeigeninhalten und für die Entgegennahme von Eingaben logisch festgelegt sind.

3. Anzeige- und Bedienvorrichtung nach Anspruch 2, **dadurch gekennzeichnet, dass** mehrere der logischen Bereiche zu einem zusammenhängenden Bereich zusammenfassbar sind.

4. Anzeige- und Bedienvorrichtung nach einem der Ansprüche 1 bis 3, **dadurch gekennzeichnet, dass** sie mehrere Anzeige/Bedieneinheiten (6) enthält, die identisch aufgebaut sind.

5. Anzeige- und Bedienvorrichtung nach einem der Ansprüche 1 bis 4, **dadurch gekennzeichnet, dass** die Anzeige/Bedieneinheiten (6) durch die Verbindung zwischen Anschlusseinrichtung (13) und Verbindungseinrichtung (16) an der Basiseinheit (7) gehaltert werden.

6. Anzeige- und Bedienvorrichtung nach einem der Ansprüche 1 bis 5, **dadurch gekennzeichnet, dass** die Anzeige/Bedieneinheiten (6) durch zusätzliche Halterungselemente an der Basiseinheit (7) gehaltert werden.

7. Anzeige- und Bedienvorrichtung nach einem der Ansprüche 1 bis 6, **dadurch gekennzeichnet, dass** Daten für die Anzeige von Ziffern, Buchstaben und grafischen Bilddarstellungen in der Anzeigensteuereinrichtung (9) der Anzeige/Bedieneinheit (6) abgespeichert sind.

8. Anzeige- und Bedienvorrichtung nach einem der Ansprüche 1 bis 7, **dadurch gekennzeichnet, dass** die Anzeige/Bedieneinheit (6) und die Konfigurationseinheit (18) dafür ausgelegt sind, dass Daten für die Anzeigeninhalte von der Konfigurationseinheit (18) an die Anzeige/Bedieneinheit (6) übertragbar und in der Anzeige/Bedieneinheit (6) speicherbar sind.

9. Anzeige- und Bedienvorrichtung nach Anspruch 8, **dadurch gekennzeichnet, dass** die Anzeige/Bedieneinheit (6) eingerichtet ist, der Konfigurationseinheit (18) zu übermitteln, welche Daten für die Anzeigeninhalte in der Anzeigensteuereinrichtung (9) gespeichert sind.

10. Anzeige- und Bedienvorrichtung nach einem der Ansprüche 1 bis 9, **dadurch gekennzeichnet, dass** die Anzeige/Bedieneinheit (6) ferner eine Buskommunikationsvorrichtung (14) umfasst , über die die Anzeigensteuereinrichtung (9) und die Eingabeauswerteeinrichtung (12) an den elektrischen Bus (15) angeschlossen sind.

11. Anzeige- und Bedienvorrichtung nach einem der Ansprüche 1 bis 10, **dadurch gekennzeichnet, dass** die Anzeige/Bedieneinheit (6) eine Touch-Screen-Anzeige/Bedieneinheit (6) ist, die eingerichtet ist, Informationen anzuzeigen und Eingaben entgegenzunehmen, und für die keine weiteren Bedienelemente vorgesehen sind.

12. Anzeige- und Bedienvorrichtung nach einem der Ansprüche 1 bis 10, **dadurch gekennzeichnet, dass** die Anzeige/Bedieneinheit (6) eine Touch-Screen-Anzeige/Bedieneinheit (6) ist, die eingerichtet ist, Informationen anzuzeigen und Eingaben entgegenzunehmen, und für die neben einem Ein/Aus-Schalter keine weiteren Bedienelemente vorgesehen sind.

13. Anzeige- und Bedienvorrichtung nach einem der Ansprüche 1 bis 12, wobei die medizintechnischen Geräte Lebenserhaltungssysteme sind.

14. Anzeige- und Bedienvorrichtung nach Anspruch 13, wobei die Lebenserhaltungssysteme Herz-Lungen-Maschinen oder Beatmungsgeräte sind.

## Claims

1. Display and operating device for medical equipment which has units that can be connected to an electrical bus (15), having:
at least one display/operating unit (6) with
∘ a display device (8)
∘ a display control device (9) which is designed to control the display device on the basis of data delivered,
∘ an input device (11),
∘ an input evaluating device (12) which is designed to evaluate the inputs made via the input device and
∘ a terminal device (13) to which the display control device (9) and the input evaluating device (12) are connected and with which the display/operating unit (6) can be connected to an electrical bus (15) and
- a base unit (7) with
∘ an electrical bus (15) for the communication of units connected to the electrical bus, to which the units of the medical equipment can be connected,
∘ connecting devices (16) which are designed in such a way that, by combining the terminal device (13) and one of the connecting devices (16), the display/operating units (6) can be connected to the electrical bus (15) in such a way that communication is possible between each of the display/operating units (6) and the units of the medical equipment connected to the electrical bus, and
∘ a configuration unit (18) which is connected to the electrical bus (15) and which is designed to send configuration data to the display/operating unit (6) via the electrical bus (15), wherein the configuration data fix the display contents of the display/operating unit (6) and the units of the medical equipment provided for communication with the display/operating unit (6);
**characterised in that**
- the display device (8) has a plurality of controllable picture elements;
- the input device (11) is transparent and disposed on the surface of the display device (8) facing towards an observer;
- the configuration data also fix the input areas of the display/operating unit (6);
- in the configuration unit (18) for each of the connecting devices (16) of the base unit (7) it is fixed which configuration data are transmitted to a display/operating unit (6) connected to the respective connecting device (16); and
- the configuration unit (18) is designed, after connection of a display/operating unit (6) to the electrical bus (15), to send to the display/operating unit (6) via the electrical bus (15) the configuration data corresponding to the connecting device (16) to which the display/operating unit (6) is connected.

2. Display and operating device according to claim 1, **characterised in that** in the display (8) of the display/operating unit (6) a plurality of areas are logically fixed for the display of display contents and for receiving inputs.

3. Display and operating device according to claim 2, **characterised in that** a plurality of the logical areas can be combined into a coherent area.

4. Display and operating device according to any of claims 1 to 3, **characterised in that** it contains a plurality of display/operating units (6) which are constructed identically.

5. Display and operating device according to any of claims 1 to 4, **characterised in that** the display/operating units (6) are supported on the base unit (7) by the connection between terminal device (13) and connecting device (16).

6. Display and operating device according to any of claims 1 to 5, **characterised in that** the display/operating units (6) are supported on the base unit (7) by additional support elements.

7. Display and operating device according to any of claims 1 to 6, **characterised in that** data for the display of figures, letters and graphic image representations are stored in the display control device (9) of the display/operating unit (6).

8. Display and operating device according to any of claims 1 to 7, **characterised in that** the display/operating unit (6) and the configuration unit (18) are designed in order that data for the display contents can be transmitted from the configuration unit (18) to the display/operating unit (6) and stored in the display/operating unit (6).

9. Display and operating device according to claim 8, **characterised in that** the display/operating unit (6) is designed to convey to the configuration unit (18) which data for the display contents are stored in the display control device (9).

10. Display and operating device according to any of claims 1 to 9, **characterised in that** the display/operating unit (6) further comprises a bus communication device (14) via which the display control device (9) and the input evaluating device (12) are connected to the electrical bus (15).

11. Display and operating device according to any of claims 1 to 10, **characterised in that** the display/operating unit (6) is a touch-screen display/operating unit (6) which is designed to display information and receive inputs and for which no further operating elements are provided.

12. Display and operating device according to any of claims 1 to 10, **characterised in that** the display/operating unit (6) is a touch-screen display/operating unit (6) which is designed to display information and receive inputs and for which, apart from an on/off switch, no further operating elements are provided.

13. Display and operating device according to any of claims 1 to 12, wherein the medical equipment is life support systems.

14. Display and operating device according to claim 13, wherein the life support systems are heart-lung machines or respirators.

## Revendications

1. Dispositif d'affichage et de commande pour appareils médico-chirurgicaux, qui présentent des unités raccordables à un bus électrique (15), avec :
au moins une unité d'affichage/de commande (6) avec
• un équipement d'affichage (8),
• un équipement de commande d'affichage (9), qui est aménagé pour commander l'équipement d'affichage sur la base de données fournies,
• un équipement d'entrée (11),
• un équipement d'évaluation d'entrée (12), qui est aménagé pour évaluer les entrées se faisant via l'équipement d'entrée et
• un équipement de raccordement (13), avec lequel l'équipement de commande d'affichage (9) et l'équipement d'évaluation d'entrée (12) sont en relation et auquel l'unité d'affichage/de commande (6) est raccordable à un bus électrique (15) et
- une unité de base (7) avec
• un bus électrique (15) pour la communication d'unités raccordées au bus électrique, auquel les unités de l'appareil médico-chirurgical sont raccordables,
• des équipements de liaison (16), qui sont aménagés de telle sorte que les unités d'affichage/de commande (6) sont raccordables au bus électrique (15) par regroupement de l'équipement de raccordement (13) et d'un des équipements de liaison (16) respectif de telle sorte qu'une communication entre chacune des unités d'affichage/de commande (6) et les unités raccordées au bus électrique (15) de l'appareil médico-chirurgical est possible, et
• une unité de configuration (18), qui est raccordée au bus électrique (15) et qui est aménagée pour transmettre des données de configuration à l'unité d'affichage/de commande (6) par le biais du bus électrique (15), les données de configuration fixant alors les contenus d'affichage de l'unité d'affichage/de commande (6) et les unités prévues pour la communication avec l'unité d'affichage/de commande (6) de l'appareil médico-chirurgical ;
**caractérisé en ce que**
- l'équipement d'affichage (8) présente une pluralité de pixels pouvant être commandés;
- l'équipement d'entrée (11) est transparent et est agencé sur la surface orientée vers un observateur de l'équipement d'affichage (8) ;
- les données de configuration fixent également les zones d'entrée de l'unité d'affichage/de commande (6) ;
- il est défini dans l'unité de configuration (18) pour chacun des équipements de liaison (16) de l'unité de base (7), quelles données de configuration sont transmises à une unité d'affichage/de commande (6) raccordée à l'équipement de liaison (16) respectif;
- l'unité de configuration (18) est aménagée pour transmettre les données de configuration correspondant à l'équipement de liaison (16), auquel l'unité d'affichage/de commande (6) est raccordée, à l'unité d'affichage/de commande (6) par le biais du bus électrique (15) après le raccordement d'une unité d'affichage/de commande (6) au bus électrique (15).

2. Dispositif d'affichage et de commande selon la revendication 1, **caractérisé en ce que** plusieurs zones pour l'affichage de contenus d'affichage et pour la réception d'entrées sont définies de manière logique dans l'affichage (8) de l'unité d'affichage/de commande (6).

3. Dispositif d'affichage et de commande selon la revendication 2, **caractérisé en ce que** plusieurs des zones logiques peuvent être regroupées en une zone liée.

4. Dispositif d'affichage et de commande selon l'une quelconque des revendications 1 à 3, **caractérisé en ce qu'**il contient plusieurs unités d'affichage/de commande (6) qui sont construites de manière identique.

5. Dispositif d'affichage et de commande selon l'une quelconque des revendications 1 à 4, **caractérisé en ce que** les unités d'affichage/de commande (6) sont maintenues sur l'unité de base (7) par la liaison entre l'équipement de raccordement (13) et l'équipement de liaison (16).

6. Dispositif d'affichage et de commande selon l'une quelconque des revendications 1 à 5, **caractérisé en ce que** les unités d'affichage/de commande (6) sont maintenues sur l'unité de base (7) par des éléments de fixation supplémentaires.

7. Dispositif d'affichage et de commande selon l'une quelconque des revendications 1 à 6, **caractérisé en ce que** des données pour l'affichage de chiffres, de lettres et de représentations d'images graphiques sont enregistrées dans l'équipement de commande d'affichage (9) de l'unité d'affichage/de commande (6).

8. Dispositif d'affichage et de commande selon l'une quelconque des revendications 1 à 7, **caractérisé en ce que** l'unité d'affichage/de commande (6) et l'unité de configuration (18) sont conçues pour pouvoir transmettre des données pour les contenus d'affichage de l'unité de configuration (18) à l'unité d'affichage/de commande (6) et pouvoir les enregistrer dans l'unité d'affichage/de commande (6).

9. Dispositif d'affichage et de commande selon la revendication 8, **caractérisé en ce que** l'unité d'affichage/de commande (6) est aménagée pour transmettre à l'unité de configuration (18) quelles données pour les contenus d'affichage sont enregistrées dans l'équipement de commande d'affichage (9).

10. Dispositif d'affichage et de commande selon l'une quelconque des revendications 1 à 9, **caractérisé en ce que** l'unité d'affichage/de commande (6) comprend en outre un équipement de communication par bus (14), par le biais duquel l'équipement de commande d'affichage (9) et l'équipement d'évaluation d'entrée (12) sont raccordés au bus électrique (15).

11. Dispositif d'affichage et de commande selon l'une quelconque des revendications 1 à 10, **caractérisé en ce que** l'unité d'affichage/de commande (6) est une unité d'affichage/de commande (6) à écran tactile, qui est aménagée pour afficher des informations et recevoir des entrées et pour laquelle aucun autre élément de commande n'est prévu.

12. Dispositif d'affichage et de commande selon l'une quelconque des revendications 1 à 10, **caractérisé en ce que** l'unité d'affichage/de commande (6) est une unité d'affichage/de commande (6) à écran tactile, qui est aménagée pour afficher des informations et recevoir des entrées et pour laquelle aucun autre élément de commande n'est prévu en plus d'un interrupteur marche/arrêt.

13. Dispositif d'affichage et de commande selon l'une quelconque des revendications 1 à 12, dans lequel les appareils médico-chirurgicaux sont des systèmes de maintien en vie.

14. Dispositif d'affichage et de commande selon la revendication 13, dans lequel les systèmes de maintien en vie sont des machines cardio-pulmonaires ou des respirateurs.
